(19) Europäisches Patentamt
European Patent Office
Office européen des brevets

(11) Veröffentlichungsnummer : **0 048 372**
**B1**

(12) # EUROPÄISCHE PATENTSCHRIFT

(45) Veröffentlichungstag der Patentschrift : **26.10.83**

(51) Int. Cl.³ : **C 07 D231/12**

(21) Anmeldenummer : **81107013.5**

(22) Anmeldetag : **07.09.81**

(54) **Verfahren zur Herstellung von Pyrazol.**

(30) Priorität : **19.09.80 DE 3035394**

(43) Veröffentlichungstag der Anmeldung : **31.03.82 Patentblatt 82/13**

(45) Bekanntmachung des Hinweises auf die Patenterteilung : **26.10.83 Patentblatt 83/43**

(84) Benannte Vertragsstaaten : **BE CH DE FR GB IT LI NL**

(56) Entgegenhaltungen :
**DE A 2 648 008**
**DE A 2 704 281**
**DE B 1 234 223**
**US A 2 515 160**

(73) Patentinhaber : **BAYER AG**
***Zentralbereich Patente, Marken und Lizenzen***
**D-5090 Leverkusen 1, Bayerwerk (DE)**

(72) Erfinder : **Lantzsch, Reinhard, Dr.**
**Heymannstrasse 32**
**D-5090 Leverkusen 1 (DE)**
Erfinder : **Ditgens, Klaus, Dr.**
**Claudiusweg 7**
**D-5600 Wuppertal 1 (DE)**
Erfinder : **Heinemann, Ulrich, Dr.**
**Thorner Strasse 22**
**D-5600 Wuppertal 2 (DE)**
Erfinder : **Thomas, Rudolf, Dr.**
**Dellbusch 269**
**D-5600 Wuppertal 2 (DE)**
Erfinder : **Weber, Erhard, Dr.**
**Claudiusweg 9**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 048 372 B1**

Jouve, 18, rue St-Denis, 75001 Paris, France

Verfahren zur Herstellung von Pyrazol

Die vorliegende Erfindung betrifft ein neues, vorteilhaftes Verfahren zur Herstellung von Pyrazol, welches als Ausgangsstoff zur Synthese von herbizid wirksamen Acetaniliden verwendet werden kann.

Es ist bereits bekannt geworden, daß man Pyrazol durch Spaltung von N-Benzolsulfonyl-pyrazolin mit einem sauren Agens, wie beispielsweise Bromwasserstoffsäure, erhält (vergleiche Chem. Ber. *92,* 1756ff (1959).

Ebenfalls bekannt ist die Herstellung von substituierten Pyrazolen durch Spaltung von entsprechenden N-Arylsulfonyl-pyrazolinen in einem basischen Medium, wie beispielsweise Kaliumhydroxid in Glykol, Natriumalkoholat in Alkohol oder Natriumhydroxid in Wasser (vergleiche Tetrahedron Letters *25,* 1665 (1963) und Zeitschrift für Chemie *5,* 456 (1956)).

Beide Verfahren haben den Nachteil, daß das unsubstituierte Pyrazol nur in unbefriedrigenden Ausbeuten erhalten wird. So liegen die Ausbeuten selbst nach sorgfältiger Aufarbeitung nur unter 50 %.

Es wurde nun gefunden, daß sich Pyrazol aus Pyrazolin-Derivaten herstellen läßt, indem man Pyrazolin-Derivate der Formel

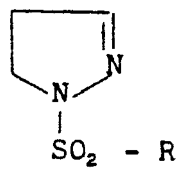

(I)

in welcher

R für gegebenenfalls substituiertes Phenyl, Alkyl oder Halogenalkyl steht,

bei Temperaturen zwischen 100 und 200 °C und bei einem Druck zwischen 1 mbar und 10 bar, gegebenenfalls in Gegenwart eines Verdünnungsmittels erhitzt.

Es ist als ausgesprochen überraschend zu bezeichnen, daß Pyrazol durch thermische Spaltung von Pyrazolin-Derivaten der Formel (I) zugänglich ist, denn aufgrund des bekannten Standes der Technik war anzunehmen, daß die betreffende Umsetzung nur bei Zusatz von Säure oder Base zum Reaktionsgemisch abläuft.

Das erfindungsgemäße Verfahren zeichnet sich durch eine Reihe von Vorteilen aus.

So bietet es eine Möglichkeit, Pyrazol auch in technischem Maßstab in einfacher Weise in hohen Ausbeuten herzustellen. Ferner sind außer dem gegebenenfalls zu verwendenden Verdünnungsmittel keinerlei Chemikalienzusätze erforderlich, welche die Umwelt belasten könnten. Schließlich fällt das Pyrazol nach der problemlosen Aufarbeitung des Reaktionsgemisches in so reiner Form an, daß keine zusätzliche Reinigung notwendig ist. Das erfindungsgemäße Verfahren stellt somit eine wertvolle Bereicherung der Technik dar.

Verwendet man beispielsweise N-Benzolsulfonylpyrazolin als Ausgangsstoff, so kann das erfindungsgemäße Verfahren durch das folgende Reaktionsschema wiedergegeben werden.

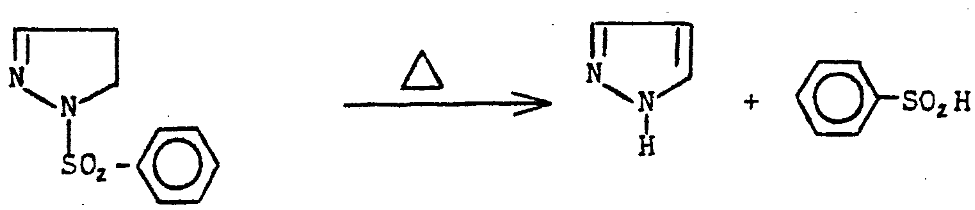

Die für das erfindungsgemäße Verfahren als Ausgangsstoffe zu verwendenden Pyrazolin-Derivate sind durch die Formel (I) allgemein definiert. In dieser Formel steht *R* vorzugsweise für gegebenenfalls durch Alkyl mit 1 bis 4 Kohlenstoffatomen oder Halogen substituiertes Phenyl, für Alkyl mit 1 bis 4 Kohlenstoffatomen sowie für Perfluoralkyl mit 1 bis 4 Kohlenstoffatomen.

Besonders bevorzugt sind diejenigen Pyrazolin-Derivate der Formel (I), bei denen *R* für gegebenenfalls durch Methyl oder Chlor substituiertes Phenyl, für Methyl oder Trifluormethyl steht.

Die Pyrazolin-Derivate der Formel (I) sind bekannt, bzw. werden in bekannter Art und Weise erhalten, indem man Pyrazolin mit den entsprechenden Sulfonsäurechloriden umsetzt (vergleiche hierzu auch die oben genannten Literaturstellen sowie die Herstellungsbeispiele). Die entsprechenden Pyrazolin-Derivate der Formel (I) können gegebenenfalls auch ohne Isolierung direkt weiter zum Pyrazol pyrolysiert werden.

Das erfindungsgemäße Verfahren wird gegebenenfalls in Gegenwart eines Verdünnungsmittels durchgeführt. Als Solventien kommen dabei vorzugsweise in Frage aromatische Kohlenwasserstoffe, wie Toluol, Xylol, Chlorbenzol oder Dichlorbenzol, insbesondere aber auch Wasser bzw. Gemische aus Wasser und einem der vorgenannten aromatischen Kohlenwasserstoffe.

Das erfindungsgemäße Verfahren kann unter vermindertem oder erhöhtem Druck durchgeführt werden. Im allgemeinen arbeitet man zwischen 1 mbar und 500 mbar, wenn kein Verdünnungsmittel verwendet wird, und zwischen Normaldruck und 10 bar bei Verwendung eines Verdünnungsmittels.

Die Reaktionstemperaturen können bei der Durchführung des erfindungsgemäßen Verfahrens in einem größeren Bereich variiert werden. Im allgemeinen arbeitet man zwischen 100 und 200 °C, vorzugsweise zwischen 140 und 180 °C.

Arbeitet man bei der Durchführung des erfindungsgemäßen Verfahrens ohne Verdünnungsmittel, so geht man im allgemeinen so vor, daß man das Ausgangsprodukt der Formel (I) in einer Destillationsapparatur unter vermindertem Druck erhitzt. Dabei destilliert das entstehende Pyrazol ab und wird in hochreinem Zustand aufgefangen.

Arbeitet man bei der Durchführung des erfindungsgemäßen Verfahrens in Gegenwart eines Verdünnungsmittels, so geht man im allgemeinen so vor, daß man Ausgangsprodukt der Formel (I) in dem jeweiligen Verdünnungsmittel löst oder suspendiert und das Reaktionsgemisch in einer verschlossenen Apparatur unter dem entstehenden Eigendruck erhitzt. Die Aufarbeitung erfolgt nach üblichen Methoden. Im allgemeinen verfährt man in der Weise, daß man das Reaktionsgemisch mit wäßrigen Alkalibasen behandelt, dann mit einem mit Wasser wenig mischbaren organischen Lösungsmittel extrahiert und dann das Lösungsmittel abdestilliert. Das anfallende Pyrazol ist bereits sehr rein und braucht normalerweise nicht mehr durch Destillation und braucht normalerweise nicht mehr durch Destillation gereinigt werden. Es ist auch möglich, das Reaktionsgemisch nach der Behandlung mit wäßriger Alkalibase einzuengen und den verbleibenden Rückstand zu destillieren.

Das nach dem erfindungsgemäßen Verfahren herstellbare Pyrazol ist ein allgemein interessanter Synthesebaustein in der organischen Chemie. Insbesondere kann Pyrazol als Ausgangsstoff zur Synthese von herbizid wirksamen Acetaniliden dienen (vgl. DE-OS 26 48 008 und DE-OS 27 04 281).

So läßt sich beispielsweise das 2,6-Diethyl-N-(pyrazol-1-yl-methyl)-chloracetanilid herstellen, indem man 2,6-Diethyl-N-chlormethyl-chloracetanilid in Gegenwart eines Verdünnungsmittels sowie in Gegenwart eines Säurebinders mit Pyrazol umsetzt. Formelmäßig läßt sich diese Synthese wie folgt wiedergeben :

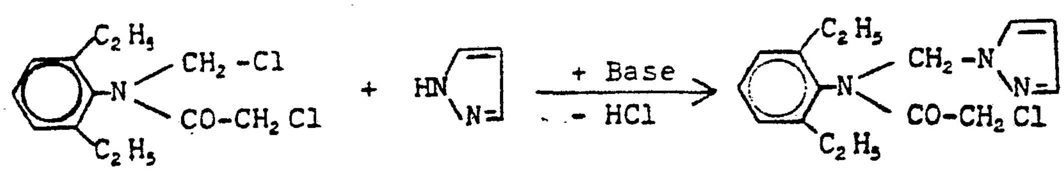


Das erfindungsgemäße Verfahren wird durch die nachfolgenden Beispiele veranschaulicht.

## Herstellungsbeispiele

### Beispiel 1

63 g (0,3 Mol) Benzolsulfonylpyrazolin werden in einer Destillationsapparatur auf 150 °C/10 Torr erhitzt. Es destilliert eine schwach gelbliche Flüssigkeit über, die nach kurzer Zeit kristallisiert. Man erhält 14,7 g (72 % der Theorie) Pyrazol vom Schmelzpunkt 69 °C.

### Beispiel 2

Entsprechend Beispiel 1 erhält man aus 14,8 g (0,1 Mol) Methansulfonylpyrazolin 4,2 g (62 % der Theorie) Pyrazol vom Schmelzpunkt 70 °C.

### Herstellung des Ausgangsproduktes

114,5 g Methansulfonsäurechlorid werden bei Raumtemperatur zu 70 g Pyrazolin und 101 g Triethylamin in 300 ml Methylenchlorid getropft. Man läßt 3 Stunden bei Raumtemperatur nachrühren und filtriert. Das Filtrat wird mit Wasser gewaschen, über Natriumsulfat getrocknet und eingeengt. Man erhält 114 g (77 % der Theorie) Methansulfonylpyrazolin vom Schmelzpunkt 62 °C.

### Beispiel 3

105 g (0,5 Mol) Benzolsulfonylpyrazolin werden in 950 ml Wasser suspendiert und eine Stunde auf 150 °C erhitzt, wobei ein Druck von 5 bar erreicht wird. Eine quantitative gaschromatographische Analyse mit innerem Standard ergibt 32 g (94,1 % der Theorie) Pyrazol. Danach läßt man abkühlen, stellt die Reaktionslösung durch Zugabe von Natronlauge schwach alkalisch und extrahiert mit Methylenchlorid im Perforator. Man erhält 30,9 g (91 % der Theorie) Pyrazol vom Schmelzpunkt 70 °C.

## Beispiel 4

65,12 g (0,44 Mol) Methansulfonylpyrazolin werden in 590 ml Wasser suspendiert und eine Stunde auf 145 °C erhitzt, wobei ein Druck von 10 bar erreicht wird. Nach dem Abkühlen stellt man mit Natronlauge schwach alkalisch. Eine quantitative gaschromatographische Analyse mit innerem Standard ergibt, daß 27,4 g (91,6 % der Theorie) Pyrazol entstanden sind. Es wird durch Extraktion mit Methylenchlorid isoliert.

## Beispiel 5

21 g (0,1 Mol) Benzolsulfonylpyrazolin werden in 190 g Xylol (Isomerengemisch) suspendiert und eine Stunde zum Sieden erhitzt. Man versetzt mit Wasser und stellt unter Rühren schwach alkalisch. Die organische Phase wird abgetrennt und die Wasserphase noch fünfmal mit Methylenchlorid ausgeschüttelt. Die vereinigten organischen Phasen werden getrocknet und destilliert. Man erhält 5,7 g (83,8 % d. Th.) Pyrazol.

## Vergleichsbeispiele zur Synthese von Pyrazol nach bekannten Methoden

## Beispiel A

21 g (0,1 Mol) Benzolsulfonylpyrazolin werden in einer Lösung von 5,4 g (0,1 Mol) Natriummethylat in 200 ml Methanol gelöst. Man erhitzt eine Stunde zum Sieden. Nach dem Abkühlen erhält man eine klare Lösung. Eine quantitative gaschromatographische Analyse mit innerem Standard ergibt, daß 1,78 g (26,2 % der Theorie) Pyrazol entstanden sind. Man verdünnt mit Wasser und extrahiert mit Methylenchlorid im Perforator. Nach dem Abdestillieren des Lösungsmittels bleibt ein Öl zurück aus dem durch Destillation (Kugelrohr) 1,1 g Pyrazol isoliert werden können.

## Beispiel B

21 g (0,1 Mol) Benzolsulfonylpyrazolin werden in 40 g 10 %iger heißer Natronlauge suspendiert, wobei eine klare Lösung entsteht. Man hält die Reaktionslösung noch kurze Zeit bei 75 °C und läßt dann abkühlen. Eine quantitative gaschromatographische Analyse mit innerem Standard ergibt, daß 3,16 g (46 % der Theorie) Pyrazol entstanden sind. Aus der klaren Lösung wird im Vakuum bei 45 °C Badtemperatur das Wasser abdestilliert; der Rückstand wird mehrmals mit Ether digeriert. Die vereinigten Etherauszüge werden über Natriumsulfat getrocknet und eingeengt. Man erhält 2,9 g Pyrazol vom Schmp. 65-66 °C.

## Beispiel C

10,5 g Benzolsulfonyl-pyrazolin werden 2 Stunden mit 60 ccm 48 proz. Bromwasserstoffsäure unter Rückfluß gekocht. Es scheidet sich Diphenyldisulfid ab, das nach dem Erkalten erstrarrt. Es wird abfiltriert; aus Alkohol Schmp. 61° (Lit.: 61). Ausb. 1,2 g (22 % d. Th., bezogen auf das Benzolsulfoderivat). Mit Zink in Salzsäure wird es zum Thiophenol reduziert.

Das Filtrat wird i. Vak. von der Bromwasserstoffsäure befreit, der weiße Rückstand mit einem Überschuß von 50 proz. Natronlauge verrieben. Man läßt darauf das zerriebene Gemisch in einen Kolben eintropfen, in dem sich etwa 50 ccm Ether befinden, und rührt heftig. Beide Teile müssen gut durchmischt werden. Dann läßt man absitzen und dakantiert den Ether; dasselbe wird nochmals mit neuem Ether wiederholt. Beide Auszüge werden vereinigt und über Natriumsulfat getrocknet: sie hinterlassen rohes Pyrazol, Schmp. 65°, Ausb. 1,6 g (47 % d. Th., bezogen auf das Benzolsulfoderivat des Pyrazolins). Aus Ligroin weiße Nadeln, Schmp. 69°.

## Beispiel für die Verwendung von Pyrazol zur Synthese eines herbizid wirksamen Acetanilids

## Beispiel I

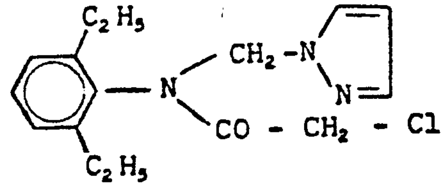

Zu 274,2 g (1 Mol) 2,6-Diethyl-N-chlormethyl-chloracetanilid in 250 ml wasserfreiem Essigester gibt man unter Rühren eine Mischung aus 68 g (1 Mol) Pyrazol und 106 g (1,05 Mol) Triethylamin in 150 ml

wasserfreiem Essigester, wobei die Temperatur auf 30 °C ansteigt. Manrührt 1 Stunde bei Raumtemperatur nach. Für die Aufarbeitung ergeben sich zwei Möglichkeiten :

a) Das Reaktionsgemisch wird filtriert, das Filtrat mit Wasser neutral gewaschen, über Natriumsulfat getrocknet und im Vakuum eingedampft. Nach einer fraktionierten Kristallisation mit Ligroin erhält man 171,2 g (56 % der Theorie) 2,6-Diethyl-N-(pyrazol-l-yl-methyl)-chloracetanilid vom Schmelzpunkt 67 °C in Form farbloser Kristalle.

b) Das Reaktionsgemisch wird auf 0 °C abgekühlt, filtriert und der Filterrückstand mit 10 ml kalltem Essigester nachgewaschen. In das Filtrat werden bei 0 bis −10 °C 50 g (1,4 Mol) trockener Chlorwasserstoff eingeleitet. Man saugt anschließend die ausgefallenen Hydrochlorid-Salze ab, wäscht mit 50 ml kaltem Essigester nach und verteilt den festen Rückstand zwischen 0,5 l Essigester und 0,5 l wäßriger Natriumhydroxid-Lösung mit einem pH-Wert von 12. Die organische Phase wird abgetrennt, zweimal mit je 0,5 l Natriumchloridlösung gewaschen, über Natriumsulfat getrocknet und im Vakuum eingedampft. Der farblose, ölige Rückstand wird mit 60 ml Benzin versetzt, wobei er kristallisiert. Man erhält 220,2 g (72 % der Theorie) 2,6-Diethyl-N-(pyrazol-l-yl-methyl)-chloracetanilid vom Schmelzpunkt 67 °C in Form farbloser Kristalle.

**Ansprüche**

1. Verfahren zur Herstellung von Pyrazol aus Pyrazolin-Derivaten, dadurch gekennzeichnet, daß man Pyrazolin-Derivate der Formel

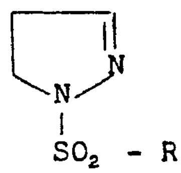

(I)

in welcher

R für gegebenenfalls substituiertes Phenyl, Alkyl oder Halogenalkyl steht,

bei Temperaturen zwischen 100 und 200 °C und bei einem Druck zwischen 1 mbar und 10 bar, gegebenenfalls in Gegenwart eines Verdünnungsmittels, erhitzt.

2. Verfahren gemäß Anspruch 1, dadurch gekennzeichnet, daß man als Ausgangsstoffe solche Pyrazolin-Derivate der Formel (I) einsetzt, in denen R für gegebenenfalls durch Alkyl mit 1 bis 4 Kohlenstoffatomen oder Halogen substituiertes Phenyl, für Alkyl mit 1 bis 4 Kohlenstoffatomen oder für Perfluoralkyl mit 1 bis 4 Kohlenstoffatomen steht.

3. Verfahren gemäß Anspruch 1, dadurch gekennzeichnet, daß man bei Temperaturen zwischen 140 °C und 180 °C arbeitet.

4. Verfahren gemäß Anspruch 1, dadurch gekennzeichnet, daß man in Abwesenheit eines Verdünnungsmittels arbeitet.

5. Verfahren gemäß Anspruch 1, dadurch gekennzeichnet, daß man in Gegenwart eines Verdünnungsmittels arbeitet.

6. Verfahren gemäß Ansprüchen 1 und 5, dadurch gekennzeichnet, daß man Toluol, Xylol, Chlorbenzol, Dichlorbenzol oder Wasser oder ein Gemisch aus Wasser und einem der vorgenannten Solventien als Verdünnungsmittel einsetzt.

**Claims**

1. Process for the preparation of pyrazole from pyrazoline derivatives, characterised in that pyrazoline derivatives of the formula

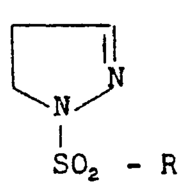

(I)

in which

R represents optionally substituted phenyl, alkyl or halogenoalkyl,

are heated at temperatures between 100 and 200 °C and under a pressure between 1 mbar and 10 bars, if appropriate in the presence of a diluent.

2. Process according to Claim 1, characterised in that those pyrazoline derivatives of the formula (I)

in which R represents phenyl which is optionally substituted by alkyl with 1 to 4 carbon atoms or halogen, or alkyl with 1 to 4 carbon atoms or perfluoroalkyl with 1 to 4 carbon atoms, are employed as the starting substances.

3. Process according to Claim 1, characterised in that the reaction is carried out at temperatures between 140 °C and 180 °C.

4. Process according to Claim 1, characterised in that it is carried out in the absence of a diluent.

5. Process according to Claim 1, characterised in that it is carried out in the presence of a diluent.

6. Process according to Claims 1 and 5, characterised in that toluene, xylene, chlorobenzene, dichlorobenzene or water or a mixture of water and one of the abovementioned solvents is employed as the diluent.

**Revendications**

1. Procédé pour la fabrication de pyrazole à partir de dérivés de pyrazoline, caractérisé en ce que l'on chauffe des dérivés de pyrazoline de formule

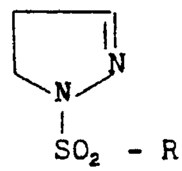

(I)

dans laquelle

R représente un groupe phényle éventuellement substitué, alkyle ou halogénoalkyle, à des températures comprises entre 100 et 200 °C et sous une pression comprise entre 1 mbar et 10 bars, éventuellement en présence d'un agent diluant.

2. Procédé selon la revendication 1, caractérisé en ce que l'on utilise comme produits de départ des dérivés de pyrazolines de formule (I) dans lesquels R représente un groupe phényle éventuellement substitué par alkyle en $C_1$-$C_4$ ou halogène, un groupe alkyle en $C_1$-$C_4$ ou un groupe perfluoroalkyle en $C_1$-$C_4$.

3. Procédé selon la revendication 1, caractérisé en ce que l'on opère à des températures comprises entre 140 et 180 °C.

4. Procédé selon la revendication 1, caractérisé en ce que l'on opère en l'absence d'un diluant.

5. Procédé selon la revendication 1, caractérisé en ce que l'on opère en présence d'un diluant.

6. Procédé selon les revendications 1 et 5, caractérisé en ce que l'on utilise comme diluants le toluène, le xylène, le chlorobenzène ou le dichlorobenzène ou bien l'eau ou un mélange d'eau ou d'un des solvants mentionnés précédemment.